(19) Europäisches Patentamt
European Patent Office
Office européen des brevets

(11) **EP 4 007 797 B1**

(12) **FASCICULE DE BREVET EUROPEEN**

(45) Date de publication et mention
de la délivrance du brevet:
**26.07.2023 Bulletin 2023/30**

(21) Numéro de dépôt: **20756932.8**

(22) Date de dépôt: **29.07.2020**

(51) Classification Internationale des Brevets (IPC):
*C09J 123/22* *(2006.01)*   *C08L 23/22* *(2006.01)*
*A61L 15/58* *(2006.01)*   *C08L 53/02* *(2006.01)*

(52) Classification Coopérative des Brevets (CPC):
(C-Sets disponibles)
**A61L 15/44; A61L 15/42; A61L 15/585;
C08L 23/22; C08L 53/025; C09J 123/22;**
C08L 2205/22                                    (Cont.)

(86) Numéro de dépôt international:
**PCT/FR2020/051395**

(87) Numéro de publication internationale:
**WO 2021/023926 (11.02.2021 Gazette 2021/06)**

(54) **COMPOSITION ELASTOMERIQUE**

ELASTOMERZUSAMMENSETZUNG

ELASTOMER COMPOSITION

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **02.08.2019 FR 1908880**

(43) Date de publication de la demande:
**08.06.2022 Bulletin 2022/23**

(73) Titulaire: **URGO RECHERCHE INNOVATION ET
DEVELOPPEMENT
21300 Chenôve (FR)**

(72) Inventeur: **GUILLAMAUD, Christelle Marie Aline
21300 Chenove (FR)**

(74) Mandataire: **Plasseraud IP
66, rue de la Chaussée d'Antin
75440 Paris Cedex 09 (FR)**

(56) Documents cités:
**EP-A1- 0 130 061     EP-A1- 3 357 517
US-A- 6 080 797       US-B1- 6 270 794**

EP 4 007 797 B1

(52) Classification Coopérative des Brevets (CPC):
(Cont.)

C-Sets
**A61L 15/585, C08L 23/18;**
**A61L 15/585, C08L 33/08;**
**A61L 15/585, C08L 33/10;**
**A61L 15/585, C08L 53/02;**
**C08L 23/22, C08L 53/02, C08L 33/20;**
**C08L 23/22, C08L 53/02, C08L 33/20, C08L 91/00;**
**C09J 123/22, C08L 53/02, C08L 33/20;**
**C09J 123/22, C08L 53/02, C08L 33/20, C08L 91/00**

**Description**

La présente invention est relative à une nouvelle composition à base de copolymères tribloc du type ABA, comportant deux blocs terminaux thermoplastiques A styrène, vitreux à température d'utilisation, et une séquence centrale élastomérique B qui est une oléfine saturée, au moins un polymère linéaire de type polyisobutène de très bas poids moléculaire et des particules d'un polymère réticulé spécifiques. La présente composition présente des propriétés d'adhésion et de tenue sur peau intéressantes s'apparentant aux propriétés obtenues au moyen de matrices à base d'élastomères de silicone. Elles présentent en outre une cohésion particulièrement intéressante.

La présente invention est également relative à un pansement comprenant une matrice obtenue à partir d'une telle composition élastomérique.

**Technique antérieure**

Les élastomères de silicone sont des composés largement connus. Ils présentent en effet des propriétés physiques et mécaniques exceptionnelles et sont utilisés dans de nombreux domaines (automobile, dispositifs médicaux, puériculture, optique, cosmétique, etc...). Dans le domaine des adhésifs, les élastomères de silicone sont particulièrement intéressants, notamment dans leur application sur la peau, les plaies, les phanères ou les muqueuses. Ce sont des adhésifs « doux », qui ne sont pas agressifs sur la peau au sens où ils sont bien tolérés, tout en présentant une bonne tenue dans le temps de l'adhérence. Ils sont en outre repositionnables, atraumatiques lors de leur retrait et donc bien tolérés par la peau, en particulier les peaux fragiles telles que la peau péri-lésionnelle. Ces adhésifs peuvent être appliqués, retirés puis appliqués à nouveau, et ce sans laisser de résidus ni provoquer de rougeurs. Cependant, les procédés de fabrication des élastomères de silicone sont assez complexes : qu'ils soient obtenus par vulcanisation à chaud ou à froid, leur procédé de fabrication doit respecter des conditions de température et d'humidité très précises. De par la nature des composants entrant dans leur composition et leur procédé d'obtention, les élastomères de silicone sont donc onéreux.

En outre, lorsqu'ils sont destinés à un usage médical, les élastomères de silicone doivent être stérilisés. Or, ces composés ne peuvent être stérilisés que par une méthode bien spécifique à l'oxyde d'éthylène, car leur exposition à tout rayonnement lumineux (constituant une technique classique de stérilisation) entraine une modification de leur structure, résultant en une dégradation de leurs propriétés adhésives.

**Résumé**

Ainsi, la présente invention propose de mettre au point une composition spécifique à base d'élastomères non siliconés présentant des propriétés d'adhésion et de tenue sur peau intéressantes, s'approchant des propriétés obtenues au moyen de compositions à base d'élastomères de silicone. De telles compositions, présentant les propriétés d'adhérence recherchées, peuvent en particulier être obtenues sans ajout de résine tackifiante. En effet, pour conférer des propriétés de collant suffisantes aux compositions à base d'élastomères non siliconés, il est généralement nécessaire d'introduire une résine tackifiante. La présence d'une telle résine confère, au toucher, un collant plus sec donc moins doux et plus agressif se traduisant par une moins bonne conformabilité et une adhérence très importante. L'ajout d'une résine peut, de plus, entrainer une instabilité de l'ensemble de la composition.

Plus précisément, il a été découvert, et ceci constitue le fondement de la présente invention, que des compositions mettant en oeuvre au moins un copolymère élastomérique tribloc spécifique, du type styrène - oléfine saturée - styrène, au moins un polymère de type polyisobutène de très bas poids moléculaire, et des particules d'un polymère réticulé spécifique, en une quantité pondérale prédéterminée, permettent de réaliser des matrices élastomériques présentant des propriétés adhésives améliorées, s'apparentant à celles obtenues avec des élastomères de silicone, tout en présentant une cohésion améliorée.

L'invention couvre ainsi également une matrice élastomérique obtenue au moyen d'une composition telle que décrite précédemment.

Les matrices élastomériques de l'invention peuvent être obtenues à partir d'un procédé de fabrication classique. Elles peuvent être stérilisées par rayonnement contrairement aux matrices mettant en oeuvre des adhésifs siliconés.

Une fois appliquées, les matrices élastomériques obtenues, pouvant être mises en oeuvre dans divers dispositifs, en particulier adhésifs, tels que par exemple des pansements, se manipulent facilement et sont résistantes, ils sont repositionnables, permettent un retrait sans douleur lorsqu'ils sont appliqués sur la peau, les muqueuses ou les phanères, et présentent une tenue dans le temps satisfaisante.

Ainsi selon un premier aspect, la présente invention a pour objet une composition comprenant :

- 2,5 à 20 % d'un copolymère tribloc styrène - oléfine saturée - styrène
- 30 à 96,5 % en poids d'un polyisobutène de poids moléculaire en nombre compris entre 700 g.mol$^{-1}$ et 3000 g.mol$^{-1}$, et

- 1 à 25 % en poids de particules d'un polymère réticulé ayant une densité de groupement carboxylate comprise entre 2,0 et 12,0 meq/g et une taille de pore moyenne comprise entre 0,005 et 1,0 $\mu$m,

les pourcentages étant exprimés en poids, par rapport au poids total de la composition.

Selon un second aspect, la présente invention a pour objet une composition comprenant :

- 4 à 12 % en poids d'un copolymère tribloc styrène - oléfine saturée - styrène,
- 30 à 70 % en poids d'un polyisobutène de poids moléculaire en nombre compris entre 700 g.mol$^{-1}$ et 3000 g.mol$^{-1}$,
- 20 à 70 % en poids d'un plastifiant, et
- 1 à 25 % en poids de particules d'un polymère réticulé ayant une densité de groupement carboxylate comprise entre 2,0 et 12,0 meq/g et une taille de pore moyenne comprise entre 0,005 et 1,0 $\mu$m,

les pourcentages étant exprimés en poids, par rapport au poids total de la composition.

En particulier, selon un mode préféré des premier et second aspects de l'invention, la composition comprend en outre 0.05 à 0.4 % en poids d'un agent antioxydant, les pourcentages étant exprimés en poids, par rapport au poids total de la composition. L'agent antioxydant permet d'avoir une composition stable dans le temps.

Selon un troisième aspect, la présente invention a pour objet une matrice élastomérique obtenue à partir d'une telle composition et un pansement comprenant ladite matrice élastomérique.

Enfin, l'invention a encore pour objet l'utilisation de particules d'un polymère réticulé ayant une densité de groupement carboxylate comprise entre 2,0 et 12,0 meq/g et une taille de pore moyenne comprise entre 0,005 et 1,0 $\mu$m pour améliorer la cohésion d'une matrice élastomérique telle que décrite précédemment.

**Description des modes de réalisation**

Copolymère tribloc styrène - oléfine saturée - styrène

La composition selon l'invention, comprend au moins un copolymère séquencé tribloc du type ABA (styrène - oléfine saturée - styrène).

Les copolymères séquencés utilisés dans le cadre de l'invention sont des copolymères triblocs du type ABA comportant deux blocs terminaux A styrène (blocs non élastomériques, vitreux à température d'utilisation, en particulier thermoplastiques) et une séquence centrale B oléfine saturée (bloc élastomérique). Ils sont notamment préparés par des techniques de polymérisation anionique ou radicalaire. Les copolymères triblocs peuvent revêtir des structures diverses : linéaire, en étoile (également dénommée radiale), branchée ou encore en peigne.

Par souci de simplicité, dans la présente description, les blocs polymériques constituant les copolymères précités sont désignés par la nature de leurs unités récurrentes. Ainsi, l'expression « bloc » ou « séquence styrène A » désigne une séquence poly(styrène) et l'expression « bloc » ou « séquence oléfine saturée » désigne une séquence poly(oléfine saturée).

Les blocs A sont donc des blocs non élastomériques styréniques (ou polystyréniques).

Les blocs B d'oléfines saturées peuvent par exemple être :

- le polyéthylène hydrogéné suivi d'un bloc polybutylène hydrogéné : le copolymère bloc a alors pour structure: polystyrène-poly(éthylène-butylène)-polystyrène et pour dénomination : SEBS ;
- le polyéthylène hydrogéné suivi d'un bloc polypropylène hydrogéné: le copolymère bloc a alors pour structure : polystyrène-poly(éthylène-propylène)-polystyrène et pour dénomination : SEPS ;
- le polyéthylène hydrogéné suivi d'un bloc polyéthylène hydrogéné puis d'une bloc polypropylène hydrogéné: le copolymère bloc a alors pour structure : polystyrène-poly(éthylène-éthylène-propylène)-polystyrène et pour dénomination : SEEPS ;
- le polyisoprène : le copolymère bloc a alors pour structure : polystyrène-polyisoprène-polystyrène, et pour dénomination : SIS ;
- le polyisoprène suivi d'un bloc polybutadiène : le copolymère bloc a alors pour structure : polystyrène-polyisoprène-polybutadiène-polystyrène, et pour dénomination : SIBS ; ou
- le polybutadiène : le copolymère bloc a alors pour structure : polystyrène-polybutadiène-polystyrène, et pour dénomination : SBS.

Selon un mode préféré de réalisation, les blocs B sont des blocs éthylène-butylène, éthylène-propylène ou éthylène-éthylène-propylène. Les copolymères blocs préférés sont ainsi choisis parmi le polystyrène-poly(éthylène-butylène)-polystyrène (SEBS), le polystyrène-poly(éthylène-propylène)-polystyrène (SEPS) et le polystyrène-poly(éthylène-éthylène-propylène)-polystyrène (SEEPS).

Dans le cadre de la présente invention, on préférera les copolymères triblocs SEBS, SEPS ou SEEPS ayant une teneur en styrène comprise entre 10 et 45 % en poids, de préférence entre 10 et 35 % en poids, par rapport au poids dudit copolymère SEBS, SEPS ou SEEPS.

Les copolymères triblocs à séquence centrale saturée sont bien connus de l'homme de l'art et sont par exemple commercialisés :

- par la société KRATON sous la dénomination KRATON® G, et en particulier les grades KRATON® G1651, KRATON® G1654, KRATON® G1652 ou KRATON® G1650 et par la société KURARAY sous les dénominations SEPTON® et en particulier les grades 8006 ou 8004 pour les copolymères séquencés poly (styrène-éthylène-butylène-styrène) (en abrégé SEBS) ;
- par la société KURARAY sous la dénomination SEPTON® pour les copolymères séquencés poly (styrène-éthylène-propylène-styrène) (en abrégé SEPS) et en particulier les grades 2005, 2006 ou 2063 et pour les polymères séquencés poly (styrène-éthylène-éthylène-propylène-styrène) (en abrégé SEEPS) et en particulier les grades 4033, 4044, 4055, 4077 ou 4099 ;
- par la société Dynasol sous la dénomination Calprène®, en particulier Calprène® H6140 et H6144 pour un copolymère séquencé SEBS.

Selon un mode préféré de réalisation, le copolymère tribloc styrène - oléfine saturée - styrène selon l'invention présente une viscosité mesurée dans une solution à 5 % (masse/masse) dans le toluène à 30°C, comprise entre 0,01 et 1 Pa.s.

Parmi les copolymères qui présentent une viscosité comprise entre 0,01 et 1 Pa.s mesurée dans une solution à 5 %, on peut citer les copolymères commercialisés par la société KRATON sous les grades KRATON® G 1651 et KRATON® G 1654 et les copolymères commercialisés par la société KURARAY sous les grades SEPTON® 4055, 4077, 4044 ou 4099.

On peut également citer les copolymères commercialisés par la société Dynasol sous les grades Calprène® H 6140 et Calprène® H6144 ou par la société TSRC sous les grades Taipol® 6151 et Taipol® 6154 et les copolymères commercialisés par la société KURARAY.

Selon un autre mode préféré de réalisation, le copolymère tribloc styrène - oléfine saturée - styrène selon l'invention présente une viscosité mesurée dans une solution à 15% (masse/masse) dans le toluène à 30°C, comprise entre 0,01 et 0,5 Pa.s.

Parmi les copolymères qui présentent une viscosité mesurée dans une solution à 15% comprise entre 0,01 et 0,5 Pa.s, on peut citer les copolymères commercialisés par la société KRATON® sous les grades KRATON® G 1650, KRATON® G 1657 et KRATON® G 1652, KRATON® G1726, et les copolymères commercialisés par la société KURARAY sous les grades SEPTON® 8076 ou 4033. On peut également citer les copolymères commercialisés par la société TSRC sous les grades Taipol® 6150 ou 6152.

Ces viscosités sont mesurées à l'aide d'un viscosimètre Brookfield modèle LVI dans une solution dans le toluène à 5 % ou 15 % masse/masse en fonction du poids moléculaire du copolymère.

De préférence, le copolymère tribloc styrène - oléfine saturée - styrène mis en oeuvre dans le cadre de la présente invention est un polystyrène-poly(éthylène-éthylène-propylène)-polystyrène (SEEPS) tel que de préférence le Septon® 4055 ou un polystyrène-poly(éthylène-butylène)- polystyrène (SEBS), tel que de préférence le KRATON® G1654 ou les Calprène® H6144 et H6140.

De façon générale, la quantité de copolymère tribloc styrène - oléfine saturée - styrène dans la composition est comprise entre 2,5 et 20 % en poids, de préférence entre 3 et 15 % en poids, préférentiellement entre 3,5 et 10 %, plus préférentiellement entre 4 et 8 % en poids rapportée au poids total de la composition.

Outre le copolymère tribloc styrène-oléfine saturée-styrène, la composition selon l'invention peut également comprendre un copolymère dibloc. De préférence, le copolymère dibloc a pour formule générale AB dans laquelle A et B sont tels que définis précédemment.

Dans le cadre de l'invention, on préfère utiliser un mélange d'un copolymère tribloc et d'un copolymère dibloc ayant les mêmes blocs A et/ou B, en raison notamment du fait que de tels mélanges sont directement disponibles commercialement et garantissent une meilleure miscibilité du mélange.

Selon une variante préférée, la teneur en copolymère dibloc dans le mélange de copolymères diblocs/triblocs styréniques est comprise entre 30 et 95 % en poids, par rapport au poids total du mélange de copolymères diblocs/triblocs styréniques.

En particulier, le mélange de copolymère dibloc/tribloc présente un taux final contrôlé de tribloc/dibloc. Une telle association présente l'avantage d'obtenir une composition présentant une meilleure capacité de dissipation et donc une meilleure adhésivité. La dissipation est la propriété de déformation irréversible de la couche adhésive. En se déformant, ce type d'adhésif consomme de l'énergie qui est dissipée dans la déformation. C'est cette propriété qui permet notamment d'avoir une bonne adhérence, en ce sens qu'il faut une énergie importante pour obtenir cette déformation qui n'est pas emmagasinée par la couche adhésive.

Parmi les mélanges de copolymère dibloc/tribloc connu de l'homme du métier, on peut citer à titre d'exemple le KRATON® G 1726 dont le taux de styrène est de 30% ou le KRATON® G 1657 commercialisé par la société KRATON dont le taux de styrène est de 13%.

En particulier, la quantité de copolymère dibloc styrène-oléfine saturée dans la composition est comprise entre 1 et 10 % en poids, de préférence entre 2 et 6 % en poids, préférentiellement entre 2,5 et 5 % en poids, rapportée au poids total de la composition.

Selon un mode particulier de réalisation, les copolymères tribloc/dibloc styréniques sont présents dans la composition en un ratio tribloc/dibloc compris entre 1 et 3, de préférence entre 1,5 et 2.

Le polyisobutène

Le polyisobutène (PIB) est un homopolymère saturé, peu réactif (faible oxydabilité), issu du monomère isobutylène. Cette polyoléfine a pour motif de répétition -[CH2-C(CH3)2]n-.

Les PIB résultent de la copolymérisation cationique de l'isobutylène (H2C=C(CH3)2, comonomère monoinsaturé) avec l'isoprène (H2C=C(CH3)-CH=CH2, diène conjugué). La réaction s'effectue en solution dans le chlorométhane à -95 °C en présence de chlorure d'aluminium (AlCl3). Les chaînes polymères contiennent environ 1 à 2 % d'unités isopréniques (cis et trans). L'enchaînement en 1,4 du diène laisse une double liaison (insaturation).

Par PIB de très bas poids moléculaire on entend, au sens de la présente invention, des PIB dont le poids moléculaire en nombre se situe entre 700 g.mol$^{-1}$ et 3000 g.mol$^{-1}$, de préférence entre 750 g.mol$^{-1}$ et 1500 g.mol$^{-1}$, plus préférentiellement entre 800 g.mol$^{-1}$ et 1400 g.mol$^{-1}$, encore plus préférentiellement entre 850 g.mol$^{-1}$ et 1300 g.mol$^{-1}$, davantage préférentiellement entre 900 g.mol$^{-1}$ et 1200 g.mol$^{-1}$.

Le poids moléculaire en nombre du PIB est mesuré par chromatographie d'exclusion stérique selon la méthode suivante :

- Solution de PIB à 2 g/L environ dans du tétrahydrofurane (THF)
- Volume injection : 100 μL
- Débit : 1 mL/min
- Détecteur : RI (indice réfraction)
- Température four : 35°C +/- 5°C
- Température RI : 40°C

Les PIB susceptibles d'être mis en oeuvre dans le cadre de la présente invention sont bien connus de l'homme du métier et disponibles commercialement, par exemple sous les dénominations commerciales suivantes :

- TER PIB® 950 commercialisé par TER France, polyisobutène présentant un poids moléculaire en nombre de 950 g.mol$^{-1}$ ;
- REWOPAL PIB® 1000 commercialisé par EVONIK, polyisobutène présentant un poids moléculaire en nombre de 1000 g.mol$^{-1}$ ;
- Glissopal V190 (également connu sous le nom de Safinol V190 ou Safic-Chem V190, présentant un poids moléculaire en nombre de 1000 g.mol$^{-1}$), V500 (présentant un poids moléculaire en nombre de 1300 g.mol$^{-1}$) ; commercialisés par BASF
- Dynapak® 190, Dynapak® 230 commercialisés par Univar, présentant des poids moléculaires en nombre de 1000 et 1050 g.mol$^{-1}$ respectivement.

Dans le cadre de la présente invention, le PIB est de préférence présent en une quantité de 45 à 97,5 % de PIB en poids, rapportée au poids total de la composition.

Idéalement, le PIB est présent en une quantité de 30 à 96,5%, de préférence de 30 à 65%, préférentiellement 40 à 60 %, plus préférentiellement de 45 à 55 % de PIB en poids par rapport au poids total de la composition. Quand on augmente la quantité de PIB, on augmente le collant (tack), mais on diminue la cohésion.

Les particules d'un polymère réticulé

Les compositions selon l'invention comprennent des particules d'un polymère réticulé ayant une densité de groupement carboxylate comprise entre 2,0 et 12,0 meq/g et une taille de pore moyenne comprise entre 0,005 et 1,0 μm.

La « taille moyenne des pores » désigne une taille moyenne exprimée en volume, dont la valeur peut être calculée par la formule 4 V/S où S est la surface spécifique et V est le volume de pores par unité de masse obtenu à partir d'une distribution de taille de pore mesurée par la méthode de compression au mercure.

La taille moyenne des pores, exprimée en volume, peut être déterminée par toute méthode connue de l'homme du métier, par exemple par porosimétrie à intrusion de mercure ou sorptométrie par adsorption d'azote.

Par exemple, la sorptométrie par adsorption d'azote peut être réalisée au moyen d'un appareil TriStar II Micromeritics couplé à un Smart VacPrep Micromeritics. Le lot à caractériser est par exemple soumis à une phase de dégazage pendant 24 heures à température ambiante puis 5 heures à 50°C. La température en cours d'essai est de -196°C, et la pression maintenue dans une gamme de $0 < P/P_0 < 0,30$ avec $P_0$ = pression de vapeur saturante de l'azote.

La porosimétrie à intrusion de mercure peut être mise en oeuvre au moyen d'une cellule de mesure pour poudre ayant un volume de $3 cm^3$ et un capillaire de volume de $0,387 cm^3$. L'analyse est réalisée en deux temps : dans un premier temps l'ensemble « pénétromètre-échantillon » est en configuration « basse pression » (mesure de 0,52 psia (vide primaire) jusqu'à 30 psia soit 2 bars) ; dans un second temps l'ensemble « pénétromètre-échantillon » est en configuration « haute pression » (mesure jusqu'à 60000psia soit 4000 bars). La taille minimale des pores accessibles est de 3 mm.

Selon un mode préféré de réalisation, les particules de polymère réticulé mises en oeuvre dans le cadre de la présente demande présentent une surface spécifique inférieure à $1 m^2/g$. La surface spécifique peut notamment être mesurée par la méthode BET d'adsorption physique, bien connue de l'homme du métier.

Selon un mode préféré de réalisation, le polymère mis en oeuvre est un polymère organique.

Le polymère mis en oeuvre présente une densité de groupement carboxylate comprise entre 2,0 et 12,0 meq/g. Le groupement carboxylate est un groupe polaire conférant au polymère les propriétés d'absorption d'humidité souhaitées.

Il n'y a pas de limitation particulière quant à la nature du sel mis en oeuvre pour la formation des groupements carboxylate. Il peut par exemple s'agir d'un sel de métal alcalin tel que Li, Na, K, Rb et Cs, de métal alcalino-terreux tel que Be, Mg, Mg, Ca, Sr et Ba, d'autres métaux tels que Cu, Zn, Al, Al, Mn, Ag, Fe, Co et Ni, NH4 et des cations organiques tels que des amines.

De préférence, le sel de carboxylate utilisé dans le cadre de la présente invention est le carboxylate de sodium.

L'introduction de groupements carboxylates peut être effectuée par toute méthode connue de l'homme du métier. Par exemple, un monomère porteur d'un groupement carboxylate peut être homopolymérisé ou copolymérisé avec d'autres monomères pour donner le polymère selon l'invention. Alternativement, un polymère porteur de groupements carboxylés peut être salifié. Alternativement encore, un polymère peut d'abord être greffé par des groupements carboxylés, lesquels seront ensuite salifiés. Ces méthodes d'introduction de groupements carboxylates sont décrites en détails dans la demande de brevet US 6 080 797.

Un exemple typique de particules de polymère réticulé selon l'invention peut être réalisé à partir d'acrylonitrile ou d'acide méthacrylique.

En particulier, le polymère réticulé peut être préparé à partir d'au moins un monomère d'acrylate d'alkyle, notamment choisi parmi les monomères d'acrylate d'isooctyle, d'acrylate de 2-éthylhexyle, d'acrylate de butyle, d'acrylate de sec-butyle, du méthyle butyle acrylate, du 4-methyl 2-pentyl acrylate, ou d'acrylate de vinyle.

Plus particulièrement, des microparticules de polymère réticulé, en particulier de polyacrylonitrile, selon l'invention peuvent être obtenues par coagulation ou par polymérisation par précipitation pour donner un agglomérat de particules de polyacrylonitrile ou un polymère d'acrylonitrile, cet agglomérat ou ce polymère subit une réticulation avec de l'hydrazine ou un dérivé d'hydrazine et enfin une hydrolyse au moins partielle des groupements nitrile résiduels de sorte à obtenir une densité de groupements carboxylate comprise entre 2,0 et 12,0 meq/g. En outre les différentes étapes de ce procédé permettent d'obtenir une taille de pore moyenne comprise entre 0,005 et 1,0 $\mu$m.

A titre illustratif, une première méthode permettant la fabrication d'un polymère réticulé selon l'invention consiste à préparer une solution de polymère à partir d'un polymère acrylonitrile et d'un solvant, à faire ensuite coaguler ladite solution dans un solvant qui n'est pas un solvant pour ledit polymère acrylonitrile pour obtenir un polymère acrylonitrile poreux, puis faire réticuler ledit polyacrylonitrile poreux avec une hydrazine, ladite réticulation étant suivie finalement d'une hydrolyse des groupements nitriles résiduels de manière à obtenir une densité de groupement carboxylate comprise entre 2,0 et 12,0 meq/g et une taille de pore moyenne comprise entre 0,005 et 1,0 $\mu$m.

Alternativement, une seconde méthode permettant la fabrication d'un polymère réticulé selon l'invention consiste à faire polymériser par précipitation un mélange de monomères contenant au moins 50% en poids d'acrylonitrile pour obtenir un polymère acrylonitrile poreux, puis réticulation dudit polyacrylonitrile poreux avec une hydrazine et hydrolyse des groupements nitriles résiduels de manière à obtenir une densité de groupement carboxylate comprise entre 2,0 et 12,0 meq/g et une taille de pore moyenne comprise entre 0,005 et 1,0 $\mu$m.

Ces procédés sont décrits plus précisément dans la demande de brevet US 6 080 797.

Néanmoins, toutes particules ayant les propriétés de densité de groupement carboxylate ou de taille de pore requises conviennent à la réalisation d'une composition selon l'invention. Une variante typique consacrerait par exemple la réalisation de telles particules à partir d'acide méthacrylique telles que décrites à l'exemple 5 du brevet US 6 080 797.

Il n'y a pas de limitation particulière quant à la forme des particules de polymère mises en oeuvre selon l'invention.

Au sens de la présente invention, le polymère réticulé selon l'invention est caractérisé par une humidité relative d'équilibre (mesurée à 20°C sous une atmosphère à 65 % d'humidité relative) comprise entre 20 et 80 %, de préférence entre 30 et 70 %.

Selon un mode particulier de réalisation, les particules de polymère réticulé selon l'invention présentent une taille moyenne comprise entre 0,1 et 100 $\mu$m, de préférence entre 0,3 et 64 $\mu$m.

Selon un autre mode particulier de réalisation, les particules d'un polymère réticulé selon l'invention présentent une masse volumique apparente comprise entre 0,1 et 1 g/cm$^3$, de préférence entre 0,2 et 0,7 g/cm$^3$.

De telles particules sont par exemple commercialisées par la société Japan Exlan Co., Ltd sous la dénomination Taftic® HU 707E, Taftic® HU 720SF ou Taftic® HU 1200P.

Elles peuvent être introduites dans les compositions selon l'invention sous forme de poudre.

La composition selon l'invention comprend 1 à 25 %, de préférence 2 à 20%, et plus préférentiellement 3 à 17 % en poids, d'un polymère réticulé ayant une densité de groupement carboxylate comprise entre 2,0 et 12,0 meq/g et une taille de pore moyenne comprise entre 0.005 et 1.0 $\mu$m, de préférence de 0,03 à 15 parties en poids.

Le plastifiant

Selon un mode particulier de réalisation, et notamment lorsque le procédé de fabrication de la matrice élastomérique se fait par voie fondue, le copolymère tribloc styrène - oléfine saturée - styrène, le PIB et les particules de polymère réticulé présents dans la composition selon l'invention, sont associés à au moins un plastifiant.

Les plastifiants susceptibles d'être utilisés sont bien connus et destinés à améliorer les propriétés d'étirement, de souplesse, d'extrudabilité ou de mise en oeuvre du copolymère tribloc styrène - oléfine saturée - styrène. On pourra, à cet effet, utiliser un ou plusieurs plastifiants si nécessaire.

D'une façon générale, en tant que plastifiants, on préférera des composés liquides, compatibles avec la séquence centrale oléfine saturée des copolymères séquencés précités.

Parmi les composés plastifiants susceptibles d'être mis en oeuvre dans les compositions selon l'invention, on citera en particulier les huiles, de préférence minérales.

Alternativement, on peut aussi utiliser des produits de synthèse à base de mélanges liquides d'hydrocarbures saturés comme par exemple les produits commercialisés par la société TOTAL sous la dénomination GEMSEAL® et en particulier le produit GEMSEAL® 60 qui est un mélange isoparaffinique issu d'une coupe pétrolière totalement hydrogénée.

Dans le cadre de la présente invention, on utilisera de préférence des huiles plastifiantes et en particulier des huiles minérales formées de composés paraffiniques ou naphténiques, ou de leurs mélanges, dans des proportions variables.

Des huiles minérales plastifiantes particulièrement préférées sont formées de mélanges de composés paraffiniques et naphténiques, et en particulier de tels mélanges dans lesquels la proportion de composés de nature paraffinique est majoritaire.

Parmi les huiles plastifiantes convenant particulièrement, on peut citer l'huile commercialisée par la société PETRO CANADA sous la référence PURETOL® 9D ou les huiles BLANDOL® et RUDOL® commercialisées par Sonneborn ou encore l'huile Pionier® 2076P ou l'huile Pionier® 7860 commercialisées par Hansen & Rosenthal.

Outre des huiles, le plastifiant peut comprendre de la vaseline. La vaseline mise en oeuvre dans les compositions de l'invention est une vaseline conforme à la Pharmacopée Française disponible commercialement. On peut citer à titre d'exemple la Vaseline Codex A® commercialisée par Aiglon.

Dans le cadre de la présente invention, le plastifiant est présent en une quantité de 20 à 70%, de préférence de 25 à 50 %, plus préférentiellement de 30 à 40 % en poids, rapportée au poids total de la composition.

Les hydrocolloïdes

Selon un mode de réalisation de l'invention, les compositions selon l'invention peuvent comprendre des particules d'hydrocolloïde.

Ces particules, lorsqu'elles sont utilisées dans une matrice élastomérique destinée à entrer en contact avec la peau ou la plaie, permettent un retrait indolore et le maintien d'un milieu humide au niveau de la plaie afin de favoriser la cicatrisation.

A cet effet, une quantité faible de particules hydrophiles d'un hydrocolloïde est ainsi soit disposée en surface de la matrice élastomérique une fois celle-ci formée soit, de préférence, dispersée de façon homogène au sein de la composition selon l'invention.

Par « hydrocolloïde » ou « particules d'hydrocolloïde », on entend désigner ici tout composé habituellement utilisé par l'homme de l'art pour son aptitude à absorber les liquides aqueux tels que l'eau, le sérum physiologique ou les exsudats d'une plaie.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses (telle que, par exemple, la BLANOSE® 7H4XFPH commercialisée par Ashland) et leurs sels de métal alcalin tels que le sodium ou le calcium, ainsi que les polymères synthétiques à base de sels de l'acide acrylique, connus sous l'appellation "superabsorbants", comme par exemple les produits commercialisés par la société CIBA Specialty Chemicals sous la dénomination SALCARE® SC91 ainsi que les mélanges de ces composés.

Certains de ces superabsorbants qualifiés de « microcolloïdes » car ils présentent une taille de particules inférieure à 10 micromètres peuvent bien entendu être également utilisés.

Les hydrocolloïdes préférés dans le cadre de la présente invention sont les sels de métal alcalin de la carboxyméthylcellulose, et en particulier la carboxyméthylcellulose de sodium (CMC).

La taille des particules d'hydrocolloïde, par exemple mesurée par granulométrie laser, est généralement comprise entre 50 et 100 µm, avantageusement de l'ordre de 80 µm.

D'une façon générale, la quantité de particules d'hydrocolloïde incorporées dans la composition selon l'invention sera avantageusement inférieure ou égale à 25 % en poids, avantageusement de 2 à 20 % en poids, de préférence de 5 à 18 % en poids, de préférence encore de 10 à 15 % en poids, rapportée au poids total de ladite composition.

Si les particules d'hydrocolloïde sont disposées en surface de la matrice élastomérique une fois celle-ci formée, leur quantité sera de préférence de l'ordre de 1 à 10 % en poids, et plus particulièrement de 2 à 5 % en poids, rapportée au poids total de ladite matrice élastomérique.

Les antioxydants

La composition selon l'invention peut également comprendre des agents antioxydants.

Par « agents antioxydants », on entend désigner ici les composés couramment employés par l'homme de l'art pour assurer la stabilité des composés entrant dans la formulation des compositions, en particulier vis-à-vis de l'oxygène, la chaleur, l'ozone ou les rayonnements ultra-violets.

Comme exemples d'agents antioxydants appropriés, on peut citer notamment les antioxydants phénoliques comme en particulier les produits commercialisés par la société BASF sous les dénominations IRGANOX® 1010, IRGANOX® 565, IRGANOX® 1076.

D'une façon générale ces agents antioxydants pourront être utilisés seuls ou en association en une quantité de l'ordre de 0,05 à 1 % en poids, de préférence de 0,05 à 0,4 % en poids, rapportée au poids total de la composition.

Dans le cadre de la présente invention, on préférera l'utilisation du produit IRGANOX® 1010 en une quantité comprise entre 0,05 et 0,4 % en poids, rapportée au poids total de la composition.

Actifs additionnels

La composition selon l'invention peut en outre comprendre une (ou plusieurs) autre(s) substance(s) active(s) permettant d'induire ou d'accélérer la cicatrisation ou pouvant avoir un rôle favorable dans le traitement de la peau ou d'une plaie.

Parmi ces substances actives, on peut citer, en particulier, à titre d'exemples :

- les agents favorisant la cicatrisation tels que le rétinol, la vitamine A, la vitamine E, la N-Acétyl Hydroxyproline, les extraits de Centella Asiatica, la papaïne, le silicone, les huiles essentielles de thym, niaouli, romarin, sauge, l'acide hyaluronique, le sucrose octasulfate de potassium, le sucralfate, l'allantoïne, la metformine ;
- les agents antibactériens tels que les sels ou complexes d'argent (tels les sulfates d'argent, les nitrates d'argent, les sulfamides d'argent ou encore les zéolites à base d'argent), les sels de zinc ou de cuivre, le métronidazole, la néomycine, les pénicillines, l'acide clavulanique, les tétracyclines, la mynocycline, la chlorotétracycline, les aminoglycosides, l'amikacine, la gentamicine, les probiotiques ;
- les antiseptiques tels que la chlorhexidine, le trichlosan, le biguanide, l'hexamidine, le thymol, le lugol, la povidone iodée, le chlorure de benzalkonium et de benzethonium ;
- les anti-douleurs tels que le paracétamol, la codéine, le dextropropoxyphène, le tramadol, la morphine et ses dérivés, les corticoïdes et leurs dérivés ;
- les anesthésiques locaux tels que la lidocaïne, la benzocaïne, la dibucaïne, le chlorhydrate de pramoxine, la bupivacaïne, la mépivacaïne, la prilocaïne, l'étidocaïne ;
- les anti-inflammatoires comme les anti-inflammatoires non stéroïdiens (AINS), l'aspirine ou acide acétylsalicylique, l'ibuprofène, le kétoprofène, le flurbiprofène, le diclofenac, l'acéclophénac, le kétorolac, le méloxicam, le piroxicam, le ténoxicam, le naproxène, l'indométacine, le naproxcinod, le nimésulid, le célécoxib, l'étoricoxib, le parécoxib, le rofécoxib, le valdécoxib, la phénylbutazone, l'acide niflumique, l'acide méfénamique.

Ces agents actifs pourront être utilisés en une quantité de l'ordre de 0,01 à 20 % en poids, de préférence de 1 à 15 % en poids, et de préférence encore de 2 à 10 % en poids, rapportée au poids total de la composition.

La présence d'hydrocolloïdes au sein de la composition favorisera le relargage de ces agents actifs.

Bien entendu, la composition selon l'invention peut aussi comprendre un ou plusieurs autres composés connus pour leur action dans la phase de détersion comme par exemple :

- des enzymes ;
- l'urée.

Adjuvants

A titre d'adjuvants susceptibles d'être utilisés dans les compositions selon l'invention, on peut citer des composés connus pour favoriser le relargage des agents actifs, comme par exemple les produits Montanox® 80 ou Sepinov® EMT 10 qui sont couramment utilisés dans les produits URGOTUL® qui incorporent des agents actifs.

Ces adjuvants pourront être utilisés en une quantité de l'ordre de 1 à 15% en poids, rapportée au poids total de la composition.

Selon un mode préféré de réalisation, la composition selon l'invention consiste en:

- 2,5 à 20 % d'un copolymère tribloc styrène - oléfine saturée - styrène
- 30 à 96,5 % en poids d'un polyisobutène de poids moléculaire en nombre compris entre 700 g.mol$^{-1}$ et 3000 g.mol$^{-1}$, et
- 1 à 25 % en poids de particules d'un polymère réticulé ayant une densité de groupement carboxylate comprise entre 2,0 et 12,0 meq/g et une taille de pore moyenne comprise entre 0,005 et 1,0 $\mu$m,

et optionnellement, de particules d'hydrocolloïde, d'agent antioxydant, et/ou d'une ou plusieurs substance(s) active(s) permettant d'induire ou d'accélérer la cicatrisation ou pouvant avoir un rôle favorable dans le traitement des plaies,

les pourcentages étant exprimés en poids, par rapport au poids total de la composition.

Selon un autre mode préféré de réalisation, la composition selon l'invention consiste en :

- 4 à 12 % en poids d'un copolymère tribloc styrène - oléfine saturée - styrène,
- 30 à 70 % en poids d'un polyisobutène de poids moléculaire en nombre compris entre 700 g.mol$^{-1}$ et 3000 g.mol$^{-1}$,
- 20 à 70 % en poids d'un plastifiant, et
- 1 à 25 % en poids de particules d'un polymère réticulé ayant une densité de groupement carboxylate comprise entre 2,0 et 12,0 meq/g et une taille de pore moyenne comprise entre 0,005 et 1,0 $\mu$m,

les pourcentages étant exprimés en poids, par rapport au poids total de la composition.

En particulier, les compositions selon l'invention sont exemptes de résines tackifiantes. Par exempte de résine tackifiante, on entend, au sens de la présente demande, que la composition comprend moins de 0,5 % en poids de résine tackifiante, en particulier moins de 0,05 % en poids, et plus préférentiellement moins de 0,005 % en poids.

Parmi les résines tackifiantes, on peut mentionner les résines polyterpènes ou terpènes modifiées, les résines de colophane, les résines hydrocarbonées, les mélanges de résines cycliques, aromatiques et aliphatiques.

Il peut par exemple s'agir de produits commerciaux tels que :

- des résines polycyclopentadiènes hydrogénées commercialisées par la société ARAKAWA Chemical Industries sous la dénomination ARKON®P,
- des résines commercialisées par la société EXXON Chemical sous la dénomination ESCOREZ® et en particulier la série des résines 5000 qui sont hydrogénées,
- une résine de synthèse formée de copolymères en C5/C9 telle que celle commercialisée par la société CRAY VALLEY sous la dénomination WINGTACK®86, ou une résine à base de polyterpène synthétique telle que celle commercialisée par la société CRAY VALLEY sous la dénomination WINGTACK® 10,
- les résines KRISTALEX® et en particulier KRISTALEX®3105SD et F100 commercialisées par la société EASTMAN, ou Sylvares® SA100 (résine à base d'alpha-méthylstyrène) par la société ARIZONA CHEMICAL, ou encore
- les résines Sukorez® de grades SU-90; SU-100; SU- 100S commercialisées par la société Kolon Industries.

Selon un mode préféré de réalisation, les compositions selon l'invention sont exemptes d'élastomères de silicones. Par exempte d'élastomères de silicones, on entend, au sens de la présente demande, que la composition comprend moins de 0,1 % en poids d'élastomères de silicones, en particulier moins de 0,01 % en poids, et plus préférentiellement moins de 0,001 % en poids.

Procédé de préparation des compositions

Les compositions selon l'invention peuvent être préparées par toute technique connue de l'homme du métier.

Selon un mode préféré de réalisation, les compositions selon l'invention peuvent être préparées par voie « solvant » ou par voie « fondue ».

Par voie solvant, on entend, au sens de la présente demande, tout procédé consistant à dissoudre le copolymère tribloc styrène - oléfine saturée - styrène dans un solvant adapté, ledit solvant étant éliminé par évaporation à l'issue du procédé de préparation de la composition.

Par voie fondue, on entend tout procédé consistant à faire fondre le copolymère tribloc styrène - oléfine saturée - styrène pour réaliser le mélange des constituants de la composition. De préférence, le mélange est réalisé dans un mélangeur ou un malaxeur.

Dans le cadre de la présente invention :

- lorsque le procédé de préparation de la composition est effectué par voie « solvant », le PIB est de préférence présent en une quantité de 80 à 97.5 %, de préférence 80 à 95 % en poids, rapportée au poids total de la composition,
- lorsque le procédé de fabrication de la matrice se fait par voie « fondue », le PIB est de préférence présent en une quantité de 45 à 97,5 %, de préférence 50 à 60 %, préférentiellement de 52 à 58 % de PIB en poids, rapportée au poids total de la composition.

Selon un mode préféré de réalisation, lorsque le procédé de préparation de la composition est effectué par voie « solvant », la composition selon l'invention consiste en :

- 2,5 à 20 % d'un copolymère tribloc styrène - oléfine saturée - styrène
- 30 à 96,5 % en poids d'un polyisobutène de poids moléculaire en nombre compris entre 700 $g.mol^{-1}$ et 3000 $g.mol^{-1}$, et
- 1 à 25 % en poids de particules d'un polymère réticulé ayant une densité de groupement carboxylate comprise entre 2,0 et 12,0 meq/g et une taille de pore moyenne comprise entre 0,005 et 1,0 $\mu$m,

les pourcentages étant exprimés en poids, par rapport au poids total de la composition.

Il est entendu que lorsque le procédé de préparation de la composition est effectué par voie « solvant », le « solvant » utilisé dans le procédé n'entre pas dans la composition.

Selon un autre mode préféré de réalisation, lorsque le procédé de préparation de la composition est effectué par voie « fondue », la composition selon l'invention consiste en :

- 4 à 12 % en poids d'un copolymère tribloc styrène - oléfine saturée - styrène,
- 30 à 70 % en poids d'un polyisobutène de poids moléculaire en nombre compris entre 700 $g.mol^{-1}$ et 3000 $g.mol^{-1}$,
- 20 à 70 % en poids d'un plastifiant, et
- 1 à 25 % en poids de particules d'un polymère réticulé ayant une densité de groupement carboxylate comprise entre 2,0 et 12,0 meq/g et une taille de pore moyenne comprise entre 0,005 et 1,0 $\mu$m,

les pourcentages étant exprimés en poids, par rapport au poids total de la composition.

Matrice élastomérique :

La présente invention a également pour objet une matrice élastomérique obtenue à partir d'une composition telle que décrite précédemment.

En particulier, la matrice élastomérique est obtenue par formation d'une couche mince, c'est-à-dire présentant une épaisseur de 50 $\mu$m à 1 mm, de préférence de 150 $\mu$m à 400 $\mu$m par calandrage, ou par coulage à chaud de ladite composition, selon des méthodes bien connues de l'homme du métier. La matrice élastomérique peut être enduite sur un support de manière à former un dépôt ajouré ou non. Ainsi, lorsque la matrice élastomérique est destinée à être appliquée sur la peau ou sur une plaie, la matrice pourra avantageusement être enduite de manière à former un dépôt ajouré afin d'obtenir la perméabilité souhaitée.

Les matrices élastomériques obtenues dans le cadre de la présente invention présentent des propriétés adhésives améliorées, s'apparentant à celles obtenues avec des élastomères de silicone.

En particulier, les matrices élastomériques selon l'invention présentent une résistance au cisaillement d'au moins 14 N, de préférence d'au moins 15 N.

La mesure de la résistance au cisaillement a pour objectif de caractériser la cohésion de la matrice, en mesurant sa résistance lorsqu'elle est soumise à un phénomène de cisaillement linéaire. Elle est réalisée sur des matrices élastomériques obtenues à l'aide d'une presse hydraulique selon le protocole suivant, dont les conditions opératoires sont détaillées en exemple :

On a préchauffé les 2 plateaux de la presse hydraulique. Sur le plateau inférieur de la presse on a déposé un film de polyester siliconé (le côté siliconé étant disposé de façon opposée au plateau inférieur). On a déposé sur cette face environ 20 g d'une des compositions décrites et on a recouvert cette dernière par un film plastique anti-adhérent, par exemple un film de polyester siliconé - fluoré (la face siliconée - fluorée étant disposée au contact de la composition). On a placé 2 cales de 1,2 mm entre les 2 films de polyester aux extrémités du plateau inférieur de la presse et on a soumis l'ensemble à une pression de 200 bars et une température de l'ordre de 90 à 100 °C.

Pour la mesure de la résistance au cisaillement, on a laissé refroidir les plaques ainsi réalisées, et on a contrôlé leurs épaisseurs avec un micromètre de manière à obtenir une maquette dont l'épaisseur de la composition est comprise entre 0,98 mm et 1,08 mm.

Les matrices élastomériques selon l'invention présentent un tack à la boucle d'au moins 15 cN/cm et une résistance au cisaillement d'au moins 14 N, de préférence d'au moins 15 N.

Bien évidemment les modes de réalisation particuliers qui viennent d'être décrits peuvent être mis en oeuvre séparément ou selon l'une quelconque de leurs combinaisons.

Les compositions selon l'invention permettent en particulier de réaliser des matrices élastomériques présentant une adhésivité acceptable et un retrait sans douleur lorsqu'elles sont appliquées sur la peau, la plaie, la muqueuse ou les phanères.

La présente invention est illustrée dans les exemples non limitatifs présentés ci-après.

**Exemples**

Préparation des compositions

Les compositions des exemples 1 à 6 ont été élaborées à l'aide des constituants suivants dans les proportions, exprimées en pourcentage en poids, mentionnées dans le tableau 1 ci-dessous.

Elastomère : Copolymère séquencé de poly (styrène-éthylène-butylène-styrène) (en abrégé SEBS) :

- KRATON® G 1654 commercialisé par Kraton Polymer
- KRATON® G 1651 commercialisé par Kraton Polymer.

Plastifiant :

- huiles minérales Pionier® 2076P ou Pionie®r 7860 commercialisées par Hansen & Rosenthal.

Antioxydant : IRGANOX® 1010 commercialisé par la société BASF.

PIB de très bas poids moléculaire :

- Safinol® V190 ou Safi-Chem® V190 (nouveau nom commercial du Safinol® V190), commercialisés par SAFIC, présentant un poids moléculaire Mn de 1000 g.mol$^{-1}$.

Particules de polymère réticulé :

Taftic® HU-720 SF de la société Japan Exlan Co., Ltd.

Fabrication de la composition par voie fondue :

Dans un mélangeur vertical, on a introduit successivement à une température de consigne de 80°C le plastifiant et le PIB et on a agité jusqu'à l'obtention d'un mélange homogène, puis les particules d'un polymère réticulé ont été ajoutées.

On a ensuite introduit sous agitation le(s) copolymère(s) et l'antioxydant, puis on a porté la température de consigne à 150°C et agité jusqu'à l'obtention d'un mélange homogène.

On a ensuite laissé refroidir, puis on a vidangé le mélangeur.

Par la suite, on a réalisé des matrices élastomériques à partir des compositions à tester, en appliquant une forte pression à l'aide d'une presse hydraulique selon le protocole suivant :

On a préchauffé à 90°C les 2 plateaux de la presse hydraulique. Sur le plateau inférieur de la presse on a déposé un film de polyuréthane (PU) et un non tissé en polypropylène (la face non tissé en polypropylène étant disposée de façon opposée au plateau inférieur, la face PU étant couvert d'un liner papier). On a déposé sur cette face environ 3,5 g d'une des compositions décrites et on a recouvert cette dernière par un film de polyester siliconé (le côté siliconé étant disposé au contact de la composition). On a placé 2 cales de 0,25 mm entre les 2 films de polyester aux extrémités du plateau inférieur de la presse et on a soumis l'ensemble à une pression de 200 bars et une température de l'ordre de 90 à 100 °C.

On a laissé refroidir les matrices ainsi réalisées, retiré le liner papier situé sur le film PU, sur la face opposée au non-tissé, et on a contrôlé leurs épaisseurs avec un micromètre de manière à obtenir une maquette dont l'épaisseur est de l'ordre de 210 à 260 $\mu$m sans le polyester siliconé.

Mesure de la résistance au cisaillement

Par la suite, on a réalisé des matrices polymériques à partir des compositions à tester, en appliquant une forte pression à l'aide d'une presse hydraulique selon le protocole suivant :

On a préchauffé les 2 plateaux de la presse hydraulique. Sur le plateau inférieur de la presse on a déposé un film de polyester siliconé (le côté siliconé étant disposé de façon opposée au plateau inférieur). On a déposé sur cette face environ 20 g d'une des compositions décrites et on a recouvert cette dernière par un film plastique anti-adhérent, par exemple un film de polyester siliconé - fluoré (la face siliconée - fluorée étant disposée au contact de la composition). On a placé 2 cales de 1,2 mm entre les 2 films de polyester aux extrémités du plateau inférieur de la presse et on a soumis l'ensemble à une pression de 200 bars et une température de l'ordre de 90 à 100 °C.

On a laissé refroidir les plaques ainsi réalisées, et on a contrôlé leurs épaisseurs avec un micromètre de manière à obtenir une maquette dont l'épaisseur de la composition est de l'ordre du mm compris entre 0,98 mm et 1,08 mm.

Cette méthode a pour objectif de caractériser la résistance de certains matériaux lorsqu'ils sont soumis à un phénomène de cisaillement linéaire.

APPAREILLAGE, MATERIELS, REACTIFS

- Dynamomètre
- Plaquettes : deux plaquettes en acier inoxydable rectangulaires par éprouvette à tester (dimension possible : 25 x 100 x 2 mm)
- Matériau de maintien : Complexe adhésif double face + Teslin SP600 commercialisé par la société PPG Teslin.
- Cales de compensation : deux cales d'épaisseur connue et identique par échantillon à tester (ou association de cales d'épaisseur permettant d'obtenir deux fois la même épaisseur). Chaque cale ou association doit être de l'épaisseur de l'échantillon à tester et du matériau de maintien de l'échantillon le cas échéant.

ECHANTILLONNAGE ET/OU CONDITIONNEMENT

Nombre d'échantillons $\geq$ 3

Conditionnement des échantillons pendant au moins 24h à 23°C $\pm$ 2 °C et 50% $\pm$ 15% d'Humidité Relative.

MODE OPERATOIRE

Préparation des éprouvettes :

Coller le matériau de maintien sur une extrémité de la première plaquette métallique de sorte qu'il recouvre la plaquette métallique sur une longueur L = 20 mm.

Puis appuyer afin de bien faire adhérer le matériau de maintien et découper le surplus au ras de la plaquette métallique.

Répéter l'opération sur la deuxième plaquette métallique (longueur du matériau de maintien identique à la première plaquette).

Découper une bande de l'échantillon à tester (d'épaisseur 1mm) de largeur l = 25 mm (largeur identique à celle des plaquettes métalliques).

Coller l'échantillon à tester sur le matériau de maintien d'une plaquette métallique, puis découper le surplus de la bande d'échantillon au ras de la plaquette métallique de sorte que l'échantillon à tester soit bien de longueur L = 20 mm, et coller sur l'échantillon la zone avec le matériau de maintien de la seconde plaquette métallique.

Mesure :

Marquer les plaquettes métalliques à la limite métal/ échantillon Appliquer un poids sur la zone à tester (zone de 20 x 25 mm) :

- 1 kg pendant 15 secondes

Fixer délicatement le dispositif avec les cales de compensation dans les mâchoires du dynamomètre (faire attention à l'épaisseur des cales de compensation, tout le dispositif doit être plan afin que la contrainte de cisaillement s'exerce bien dans le plan vertical).

Tester l'éprouvette :

- 45 secondes après avoir retiré le poids

Procéder à l'essai de cisaillement jusqu'à la rupture de l'éprouvette à la vitesse :

$$- v = 10 \ mm.min^{-1} \pm 0,5 \ mm.min^{-1}$$

Enregistrer la courbe force/ déplacement
Vérifier que l'échantillon n'a pas glissé sur les plaquettes à l'aide des marques réalisées précédemment
Vérifier qu'il s'agit bien d'une rupture de cohésion.

EXPRESSION DES RESULTATS :

Le résultat s'exprime sous la forme de la résistance nécessaire à la rupture de l'éprouvette en N (à $10^{-1}$ près).
Les matrices élastomériques selon l'invention présentent une résistance au cisaillement d'au moins 14 N, de préférence d'au moins 15 N.

[Tableau 1]

| Composé (%) | Exemple 1 | Exemple 2 | Exemple 3 | Exemple 4 |
|---|---|---|---|---|
| Safinol® V190 | 55 | 52,2 | 49,5 | 46,7 |
| Kraton® G1654 | 6 | 5,7 | 5,4 | 5,1 |
| Pionier® 7860 | 38,8 | 36,9 | 34,9 | 33 |
| Taftic® HU-720SF | | 5 | 10 | 15 |
| Irganox® 1010 | 0,2 | 0,2 | 0,2 | 0,2 |
| Résistance au cisaillement | 13,53 | 17,12 | 21,55 | 23,40 |
| Force rupture (N) | | | | |

Les matrices élastomériques selon l'invention présentent une excellente cohésion par le biais d'une résistance au cisaillement d'au moins 14 N, de préférence d'au moins 15 N.
La matrice obtenue au moyen de la composition de l'exemple 1 (comparatif) ne comprend pas de particules de polymère réticulé Taftic® HU-720SF et présente une moins bonne résistance au cisaillement, ce qui se traduit par une moins bonne cohésion.

**Revendications**

1. Composition comprenant :

   - 2,5 à 20 % d'un copolymère tribloc styrène - oléfine saturée - styrène
   - 30 à 96,5 % en poids d'un polyisobutène de poids moléculaire en nombre compris entre 700 g.mol$^{-1}$ et 3000 g.mol$^{-1}$, et
   - 1 à 25 % en poids de particules d'un polymère réticulé ayant une densité de groupement carboxylate comprise entre 2,0 et 12,0 meq/g et une taille de pore moyenne comprise entre 0,005 et 1,0 $\mu$m déterminée comme indiqué dans la description,

   les pourcentages étant exprimés en poids, par rapport au poids total de la composition.

2. Composition selon la revendication 1, dans laquelle la quantité en copolymère tribloc styrène - oléfine saturée - styrène est comprise entre 3 et 15 % en poids, préférentiellement entre 3,5 et 10 %, plus préférentiellement entre 4 et 8 % en poids, rapportée au poids total de la composition.

3. Composition selon l'une quelconque des revendications 1 ou 2, dans laquelle le copolymère tribloc du type styrène-oléfine saturée-styrène est un SEBS ou un SEEPS.

4. Composition selon l'une quelconque des revendications 1 à 3, dans laquelle le PIB est présent en une quantité de 30 à 65%, préférentiellement 40 à 60 %, plus préférentiellement de 45 à 55 % de PIB en poids, rapportée au poids total de la composition.

5. Composition selon l'une quelconque des revendications 1 à 4, dans laquelle le poids moléculaire en nombre du PIB se situe entre entre 700 g.mol$^{-1}$ et 3000 g.mol$^{-1}$, de préférence entre 750 g.mol$^{-1}$ et 1500 g.mol$^{-1}$, plus préférentiellement entre 800 g.mol$^{-1}$ et 1400 g.mol$^{-1}$, encore plus préférentiellement entre 850 g.mol$^{-1}$ et 1300 g.mol$^{-1}$, davantage préférentiellement entre 900 g.mol$^{-1}$ et 1200 g.mol$^{-1}$.

6. Composition selon l'une quelconque des revendications 1 à 5, dans laquelle les particules de polymère réticulé présentent une taille moyenne comprise entre 0,1 et 100 $\mu$m, de préférence entre 0,3 et 64 $\mu$m.

7. Composition selon l'une des revendications précédentes, dans laquelle le polymère réticulé est préparé à partir d'acrylonitrile ou d'acide méthacrylique.

8. Composition selon l'une quelconque des revendications 1 à 7, dans laquelle le polymère réticulé est préparé à partir d'au moins un monomère d'acrylate d'alkyle, notamment choisi parmi les monomères d'acrylate d'isooctyle, d'acrylate de 2-éthylhexyle, d'acrylate de butyle, d'acrylate de sec-butyle, du méthyle butyle acrylate, du 4-methyl 2-pentyl acrylate, ou d'acrylate de vinyle.

9. Composition comprenant :

   - 4 à 12 % en poids d'un copolymère tribloc styrène - oléfine saturée - styrène,
   - 30 à 70 % en poids d'un polyisobutène de poids moléculaire en nombre compris entre 700 g.mol$^{-1}$ et 3000 g.mol$^{-1}$,
   - 20 à 70 % en poids d'un plastifiant, et
   - 1 à 25 % en poids de particules d'un polymère réticulé ayant une densité de groupement carboxylate comprise entre 2,0 et 12,0 meq/g et une taille de pore moyenne comprise entre 0,005 et 1,0 $\mu$m déterminée comme indiqué dans la description,

   les pourcentages étant exprimés en poids, par rapport au poids total de la composition.

10. Composition selon la revendication 9, dans laquelle le plastifiant est présent en une quantité de 20 à 70 %, préférentiellement de 25 à 50 %, plus préférentiellement de 30 à 40 % en poids, rapportée au poids total de la composition.

11. Composition selon l'une quelconque des revendications 9 ou 10, dans laquelle le plastifiant est une huile, de préférence une huile minérale.

**12.** Composition selon l'une des revendications précédentes, **caractérisée en ce qu'**elle comprend une ou plusieurs substance(s) active(s) permettant d'induire ou d'accélérer la cicatrisation ou pouvant avoir un rôle favorable dans le traitement des plaies, en particulier en une quantité comprise entre 0,01 et 20 % en poids, de préférence entre 1 et 15 % en poids, rapportée au poids total de la composition.

**13.** Matrice élastomérique, **caractérisée en ce qu'**elle est obtenue à partir d'une composition selon l'une des revendications 1 à 12, de préférence par formation d'une couche présentant une épaisseur de 50 μm à 1 mm, de préférence de 150 μm à 400 μm par calandrage, ou par coulage à chaud de ladite composition.

**14.** Matrice élastomérique selon la revendication 13, **caractérisée en ce qu'**elle se présente sous forme d'un dépôt ajouré.

**15.** Dispositif, de préférence pansement, **caractérisé en ce qu'**il comprend une matrice élastomérique l'une des revendications 13 ou 14.

**16.** Utilisation de particules d'un polymère réticulé ayant une densité de groupement carboxylate comprise entre 2,0 et 12,0 meq/g et une taille de pore moyenne comprise entre 0,005 et 1,0 μm pour améliorer la cohésion d'une matrice élastomérique obtenue à partir d'une composition selon l'une des revendications 1 à 12.

**Patentansprüche**

**1.** Zusammensetzung, umfassend:

- 2,5 bis 20 % eines Triblock-Copolymers Styrol-gesättigtes Olefin-Styrol,
- 30 bis 96,5 Gew.-% eines Polyisobutens mit einer Molekulargewichtszahl zwischen 700 g.mol$^{-1}$ und 3000 g.mol$^{-1}$, und
- 1 bis 25 Gew.-% Partikel eines vernetzten Polymers mit einer Carboxylatgruppendichte zwischen 2,0 und 12,0 meq/g und einer mittleren Porengröße zwischen 0,005 und 1,0 μm, gemäß der Beschreibung bestimmt,

wobei die Prozentangaben in Gewichtsprozent, bezogen auf das Gesamtgewicht der Zusammensetzung, angegeben sind.

**2.** Zusammensetzung nach Anspruch 1, in der die Menge des Triblock-Copolymers Styrol-gesättigtes Olefin-Styrol zwischen 3 und 15 Gew.-%, bevorzugt zwischen 3,5 und 10 Gew.-%, stärker bevorzugt zwischen 4 und 8 Gew.-%, bezogen auf das Gesamtgewicht der Zusammensetzung, beträgt.

**3.** Zusammensetzung nach einem der Ansprüche 1 oder 2, in der das Triblock-Copolymer vom Typ Styrol-gesättigtes Olefin-Styrol ein SEBS oder ein SEEPS ist.

**4.** Zusammensetzung nach einem der Ansprüche 1 bis 3, in der das PIB in einer Menge von 30 bis 65 %, bevorzugt 40 bis 60 %, stärker bevorzugt 45 bis 55 Gew.-% PIB, bezogen auf das Gesamtgewicht der Zusammensetzung, vorliegt.

**5.** Zusammensetzung nach einem der Ansprüche 1 bis 4, in der die Molekulargewichtszahl des PIB zwischen 700 g.mol$^{-1}$ und 3000 g.mol$^{-1}$, bevorzugt zwischen 750 g.mol$^{-1}$ und 1500 g.mol$^{-1}$, stärker bevorzugt zwischen 800 g.mol$^{-1}$ und 1400 g.mol$^{-1}$, noch stärker bevorzugt zwischen 850 g.mol$^{-1}$ und 1300 g.mol$^{-1}$, am stärksten bevorzugt zwischen 900 g.mol$^{-1}$ und 1200 g.mol$^{-1}$ liegt.

**6.** Zusammensetzung nach einem der Ansprüche 1 bis 5, in der die vernetzten Polymerpartikel eine mittlere Größe zwischen 0,1 und 100 μm, bevorzugt zwischen 0,3 und 64 μm, aufweisen.

**7.** Zusammensetzung nach einem der vorhergehenden Ansprüche, in der das vernetzte Polymer aus Acrylnitril oder Methacrylsäure hergestellt ist.

**8.** Zusammensetzung nach einem der Ansprüche 1 bis 7, in der das vernetzte Polymer aus wenigstens einem Alkylacrylatmonomer hergestellt ist, insbesondere gewählt aus den Monomeren von Isooctylacrylat, 2-Ethylhexylacrylat, Butylacrylat, Seclbutylacrylat, Methylbutylacrylat, 4-Methyl-2-pentylacrylat oder Vinylacrylat.

**9.** Zusammensetzung, umfassend:

- 4 bis 12 Gew.-% eines Triblock-Copolymers Styrol-gesättigtes Olefin-Styrol,
- 30 bis 70 Gew.-% eines Polyisobutens mit einer Molekulargewichtszahl zwischen 700 g.mol$^{-1}$ und 3000 g.mol$^{-1}$,
- 20 bis 70 Gew.-% eines Weichmachers und
- 1 bis 25 Gew.-% Partikel eines vernetzten Polymers mit einer Carboxylatgruppendichte zwischen 2,0 und 12,0 meq/g und einer mittleren Porengröße zwischen 0,005 und 1,0 $\mu$m, gemäß der Beschreibung bestimmt,

wobei die Prozentangaben in Gewichtsprozent, bezogen auf das Gesamtgewicht der Zusammensetzung, angegeben sind.

**10.** Zusammensetzung nach Anspruch 9, in der der Weichmacher in einer Menge von 20 bis 70 Gew.-%, bevorzugt von 25 bis 50 Gew.-%, stärker bevorzugt von 30 bis 40 Gew.-%, bezogen auf das Gesamtgewicht der Zusammensetzung, vorliegt.

**11.** Zusammensetzung nach einem der Ansprüche 9 oder 10, in der der Weichmacher ein Öl, bevorzugt ein Mineralöl, ist.

**12.** Zusammensetzung nach einem der vorhergehenden Ansprüche, **dadurch gekennzeichnet, dass** sie einen oder mehrere Wirkstoffe enthält, die die Wundheilung induzieren oder beschleunigen können oder eine günstige Rolle bei der Behandlung von Wunden spielen können, insbesondere in einer Menge zwischen 0,01 und 20 Gew.-%, bevorzugt von 1 bis 15 Gew.-%, bezogen auf das Gesamtgewicht der Zusammensetzung.

**13.** Elastomermatrix, **dadurch gekennzeichnet, dass** sie aus einer Zusammensetzung nach einem der Ansprüche 1 bis 12 erhalten wird, bevorzugt durch Bildung einer Schicht mit einer Dicke von 50 $\mu$m bis 1 mm, bevorzugt von 150 $\mu$m bis 400 pm durch Kalandrieren oder durch Heißgießen der Zusammensetzung.

**14.** Elastomermatrix nach Anspruch 13, **dadurch gekennzeichnet, dass** sie in Form einer durchbrochenen Ablagerung vorliegt.

**15.** Vorrichtung, bevorzugt Wundverband, **dadurch gekennzeichnet, dass** sie eine Elastomermatrix nach einem der Ansprüche 13 oder 14 umfasst.

**16.** Verwendung von Partikeln eines vernetzten Polymers mit einer Carboxylatgruppendichte zwischen 2,0 und 12,0 meq/g und einer mittleren Porengröße zwischen 0,005 und 1,0 $\mu$m zur Verbesserung der Kohäsion einer Elastomermatrix, die aus einer Zusammensetzung nach einem der Ansprüche 1 bis 12 erhalten wird.

**Claims**

**1.** Composition comprising:

- 2.5 to 20% of a styrene - saturated olefin - styrene triblock copolymer
- 30 to 96.5% by weight of a polyisobutene with a number molecular weight of between 700 g.mol$^{-1}$ and 3000 g.mol$^{-1}$, determined as indicated in the description and
- 1 to 25% by weight of particles of a crosslinked polymer having a carboxylate group density of between 2.0 and 12.0 meq/g and an average pore size of between 0.005 and 1.0 $\mu$m, determined as indicated in the description,

the percentages being expressed by weight, relative to the total weight of the composition.

**2.** Composition according to claim 1, in which the amount of styrene - saturated olefin - styrene triblock copolymer is between 3 and 15% by weight, preferably between 3.5 and 10%, more preferably between 4 and 8% by weight, relative to the total weight of the composition.

**3.** Composition according to any one of claims 1 or 2, in which the triblock copolymer of the styrene - saturated olefin - styrene type is a SEBS or a SEEPS.

**4.** Composition according to any one of claims 1 to 3, in which the PIB is contained in an amount of 30 to 65%, preferably 40 to 60%, more preferably 45 to 55% of PIB by weight, relative to the total weight of the composition.

5. Composition according to any one of claims 1 to 4, in which the number molecular weight of the PIB is between 700 g.mol$^{-1}$ and 3000 g.mol$^{-1}$, preferably between 750 g.mol$^{-1}$ and 1500 g.mol$^{-1}$, more preferably between 800 g.mol$^{-1}$ and 1400 g.mol$^{-1}$, even more preferably between 850 g.mol$^{-1}$ and 1300 g.mol$^{-1}$, more preferably between 900 g.mol$^{-1}$ -1 and 1200 g.mol$^{-1}$.

6. Composition according to any one of claims 1 to 5, in which the particles of crosslinked polymer have an average size of between 0.1 and 100 $\mu$m, preferably between 0.3 and 64 $\mu$m.

7. Composition according to one of the preceding claims, in which the crosslinked polymer is prepared from acrylonitrile or methacrylic acid.

8. Composition according to any one of claims 1 to 7, in which the crosslinked polymer is prepared from at least one alkyl acrylate monomer, in particular chosen from isooctyl acrylate, 2-ethylhexyl acrylate, butyl acrylate, sec-butyl acrylate, methyl butyl acrylate, 4-methyl 2-pentyl acrylate, or vinyl acrylate monomers.

9. Composition comprising:

   - 4 to 12% by weight of a styrene - saturated olefin - styrene triblock copolymer,
   - 30 to 70% by weight of a polyisobutene with a number molecular weight of between 700 g.mol$^{-1}$ and 3000 g.mol$^{-1}$, determined as indicated in the description,
   - 20 to 70% by weight of a plasticizer, and
   - 1 to 25% by weight of particles of a crosslinked polymer having a carboxylate group density of between 2.0 and 12.0 meq/g and an average pore size of between 0.005 and 1.0 $\mu$m, determined as indicated in the description,

   the percentages being expressed by weight, relative to the total weight of the composition.

10. Composition according to claim 9, in which the plasticizer is contained in an amount of 20 to 70%, preferably 25 to 50%, more preferably 30 to 40% by weight, relative to the total weight of the composition.

11. Composition according to any one of claims 9 or 10, in which the plasticizer is an oil, preferably a mineral oil.

12. Composition according to one of the preceding claims, **characterized in that** it comprises one or several active substance(s) making it possible to induce or accelerate cicatrization or which can have a favorable role in wound treatment, in particular in an amount of between 0.01 and 20% by weight, preferably between 1 and 15% by weight, relative to the total weight of the composition.

13. Elastomeric matrix, **characterized in that** it is obtained from a composition according to any one of claims 1 to 12, preferably by forming a layer having a thickness of 50 $\mu$m to 1 mm, preferably of 150 $\mu$m to 400 $\mu$m by calendering, or by hot casting of said composition.

14. Elastomeric matrix according to claim 13, **characterized in that** it is in the form of an openwork deposit.

15. Device, preferably a dressing, **characterized in that** it comprises an elastomeric matrix according to one of claims 13 or 14.

16. Use of particles of a crosslinked polymer having a carboxylate group density between 2.0 and 12.0 meq/g and an average pore size between 0.005 and 1.0 $\mu$m to improve the cohesion of an elastomeric matrix obtained from a composition according to one of claims 1 to 12.

**EP 4 007 797 B1**

## RÉFÉRENCES CITÉES DANS LA DESCRIPTION

**Documents brevets cités dans la description**

- US 6080797 A